# EUROPÄISCHE PATENTSCHRIFT

(11) **EP 3 769 707 B1**
(45) Veröffentlichungstag und Bekanntmachung des Hinweises auf die Patenterteilung: **14.01.2026**
(21) Anmeldenummer: 19187863.6
(22) Anmeldetag: 23.07.2019
(51) Int. Cl.: A61B 18/04, A61B 18/00

(54) **ELEKTRODENANORDNUNG**
ELECTRODE ASSEMBLY
ENSEMBLE D'ÉLECTRODE

(43) Veröffentlichungstag der Anmeldung: 27.01.2021
(73) Patentinhaber: Erbe Elektromedizin GmbH, 72072 Tübingen (DE)
(72) Erfinder: BRANDT,Tjark, 72072 Tübingen (DE); SCHNITZLER, Uwe, 72074 Tübingen (DE)
(74) Vertreter: Rüger Abel Patentanwälte PartGmbB

(56) Entgegenhaltungen:
- EP-B1- 1 684 653
- DE-A1- 10 030 111
- DE-A1- 102017 127 976
- US-A- 5 207 675
- US-A1- 2008 039 834

## Beschreibung

Die Erfindung betrifft eine Elektrodenanordnung für ein elektrochirurgisches Instrument, insbesondere für ein Instrument zur Plasmakoagulation von biologischem Gewebe.

Instrumente zur Gewebekoagulation sind aus verschiedenen Druckschriften so wie aus der Praxis bekannt. Es wird dazu auf die DE 10 2011 116 678 A1 sowie auf die DE 699 28 370 T2 verwiesen. Beide Druckschriften offenbaren Instrumente mit Elektroden, die zum Beispiel ringförmig ausgebildet sind und aus einem geeigneten Material, wie u.a. auch Wolfram, bestehen können, das sich durch seine Hitzebeständigkeit auszeichnet.

Weiter ist aus der DE 100 30 111 A1 ein Plasmakoagulationsinstrument mit einem schlauchartigen Instrumentenkörper bekannt, in dessen Lumen eine sechseckige, aus einem Metall bestehende Elektrode angeordnet ist. Diese ist mit einer elektrischen Zuleitung verbunden, um die an dem distalen Ende des Schlauchs angeordnete Elektrode mit HF-Spannung versorgen zu können. Von der am distalen Ende der plättchenförmigen Elektrode ausgebildeten Spitze geht eine elektrische Entladung aus, wodurch eine Plasmastrom, insbesondere ein Edelgas-Plasmastrom erzeugt werden kann. Der die Elektrode umspülende Gasstrom dient zugleich der Abführung von Wärme aus der Elektrode, wodurch deren übermäßige Erhitzung vermieden werden soll. Durch die Wärmeabfuhr soll außerdem eine Minimierung des Abbrandverhaltens am Entladungsabschnitt der Elektrode erreicht und dadurch die Lebensdauer des Instruments erhöht werden. Ein ähnliches Instrument geht aus der DE 10 2017 127 976 A1 hervor, die einen hohlzylindrischen Elektrodenhalter mit einer den zentralen Durchgang diametral durchquerenden Wand aufweist. In der Wand ist mittig eine Wolframelektrode gefasst. Zur Verringerung der Wärmeübertragung auf die Wand ist zwischen der Wand und der Spitze der Elektrode in der Elektrode eine Kerbe angebracht, die den zur Wärmeübertragung zur Verfügung stehenden Querschnitt mindert.

Eine effiziente Kühlung des Elektrodenplättchens durch den Gasstrom erfordert aber einen hohen Gasfluss, was nicht immer gewünscht ist.

Aus der WO 2005/046495 A1 ist es darüber hinaus bekannt, eine Zündelektrode aus einem Wolframdraht zu verwenden, dessen Ende am distalen Ende eines ansonsten schlauch- oder rohrförmigen Instrumentenkörpers platziert ist. Der in dem Lumen dieses Körpers angeordnete Wolframdraht ist in einem gewissen Abstand zu seinem distalen Ende von einem Plättchen gehalten, an dem er befestigt ist und das zu seiner Kühlung dient. Allerdings ist die Wärmeabfuhr von dem Wolframdraht durch die Übergangsstelle zwischen dem Plättchen und dem Wolframdraht behindert.

Durch Elektrodenabbrand können Partikel, insbesondere Metallpartikel, in den Funken und/oder den Plasmastrom und somit letztendlich in lebendes Gewebe geraten, was zunehmend abgelehnt wird. Es ist deswegen Aufgabe der Erfindung, ein Konzept anzugeben, mit dem sich der Materialabtrag der verwendeten Elektrode eines elektrochirurgischen Instruments im Einsatz verringern lässt.

Diese Aufgabe wird mit der Elektrodenanordnung nach Anspruch 1 gelöst:

Die erfindungsgemäße Elektrodenanordnung weist eine Elektrode auf, an der eine distal orientierte Spitze ausgebildet ist. Der Elektrodenquerschnitt nimmt in proximaler Richtung von der Spitze weg zu. In Verbindung mit dem weiteren Merkmal, wonach die Elektrode aus einem Material oder einer Materialkombination besteht, deren Wärmeleitfähigkeit größer als 20W/(m*K) ist, lässt sich damit ein sehr stark reduzierter Elektrodenabbrand erreichen.

Der Elektrodenquerschnitt kann sich ausgehend von der distalen Spitze stufenartig oder auch kontinuierlich erhöhen, bis die Elektrode die Wandung des Lumens berührt, in dem sie angeordnet ist. Findet die Querschnittsvergrößerung von der Spitze zu einem proximalen Teil der Elektrode stufenweise statt, können dazu ein oder mehrere Stufen vorgesehen sein. Die Spitze der Elektrode (und der an die Spitze angrenzende Flankenbereich der Elektrode) ist diejenige Stelle, von der typischerweise ein Funken oder ein Plasmastrom ausgeht. Die Spitze und der sich unmittelbar daran anschließende Teil der Elektrode bildet somit denjenigen Bereich in dem sich der Entladungsfußpunkt der elektrischen Entladung befindet. An diesem Fußpunkt findet eine Stromkonzentration statt, die zugleich eine Wärmequelle bildet. Durch beide Maßnahmen, nämlich die Querschnittszunahme der Elektrode in proximaler Richtung und durch Nutzung eines Materials oder einer Materialkombination für die Elektrode, deren Wärmeleitfähigkeit größer als 20W/(m*K) ist, wird an dem Elektrodenfußpunkt entstehende Wärme weit wirksamer dissipiert als es bislang bei Verwendung von Edelstahlelektroden der gleichen Bauform der Fall war. Die erfindungsgemäße Elektrode zeichnet sich somit insbesondere dadurch aus, dass deren Wärmeleitfähigkeit von der Spitze, gemessen in proximaler Richtung und/oder gemessen quer zur proximalen Richtung, größer als die Wärmeleitfähigkeit von Edelstahl ist. Vorzugsweise ist die Wärmeleitfähigkeit λ der Elektrode größer als 27W/(m*K) weiter vorzugsweise größer als 50W/(m*K), größer als 100W/m*K, größer als 200W/(m*K), größer als 300W/(m*K) und insbesondere größer als 400W/ (m*K).

Die Kombination einer Elektrodengeometrie, bei der Elektrodenquerschnitt von der Spitze in proximaler Richtung weg zunimmt, wobei die Elektrode aus hochwärmeleitfähigem Material oder einer hochwärmeleitfähigen Materialkombination ausgebildet ist, ermöglich die Nutzung einer Spitze mit besonders geringem Rundungsradius, der insbesondere kleiner sein kann als 1/10 der maximalen Querabmessung der Elektrode. Dadurch wird an der Elektrodenspitze eine hohe Feldstärke erreicht, die auch bei niedrigen HF-Spannungen und -Strömen zur Ausbildung eines Funkens und eines Plasmas führen kann. Die Elektrode ist dann besonders zündwillig.

Die Elektrode ist plättchenförmig ausgebildet, wobei die Erhöhung des Elektrodenquerschnitts durch eine entlang der Axialrichtung in proximaler Richtung von der Spitze weg-zunehmende Querabmessung der Elektrode erreicht wird. Diese Querabmessung kann kontinuierlich zunehmen, wodurch eine besonders gute Wärmeableitung erreicht wird. Die kontinuierliche Querschnitts- und Querabmessungszunahme kann erreicht werden, indem sich an die Spitze der Elektrode stufenlos ausgebildete Kanten anschließen.

Die Elektrode ist als Plättchen ausgebildet das zwei Flachseiten aufweist, die durch Schmalseiten miteinander verbunden sind. Zwischen den Schmalseiten und den Flachseiten können Kanten ausgebildet sein. Eine solche Elektrode kann beispielsweise als Blechzuschnitt bereitgestellt werden.

Als Elektrodenmaterial eignen sich insbesondere Metalle mit hoher Wärmeleitfähigkeit, wie Silber, Kupfer, Wolfram, Hartmetall (z.B. Wolframkarbid-Sintermetall) oder ähnliches. Die hohe Wärmeleitfähigkeit verhindert einen Wärmestau an der Spitze der Elektrode und erleichtert ein Abfließen der Wärme in dem gesamten Elektrodenkörper und somit auch eine Erleichterung der Wärmeabgabe an den Gasstrom. Damit genügt ein relativ geringer Gasstrom zur Kühlung der Elektrode.

Gemäß der Erfindung besteht die Elektrode aus einer Materialkombination, die dadurch gebildet ist, dass die Elektrode aus einem Grundkörper besteht, der mindestens eine Fläche aufweist, an der eine Wärmeableiteinrichtung angebracht ist. Die Wärmeableiteinrichtung erstreckt sich dabei in distaler Richtung vorzugsweise bis zu der Spitze der Elektrode zumindest bis an einen Bereich, der im Betrieb von dem Entladungsfußpunkt eingenommen wird. Die dort entstehende Wärme kann somit unmittelbar auf die Wärmeableiteinrichtung übertragen werden, ohne dass eine Wärmeübertragung von der Elektrode auf die Wärmeableiteinrichtung nötig wäre. Die Wärmeableiteinrichtung steht mit anderen Worten in direktem Kontakt mit der Wärmequelle hier in Gestalt des Entladungsfußpunkts. In proximaler Richtung erstreckt sich die Wärmeableiteinrichtung vorzugsweise mindestens bis in einen Bereich der Elektrode, in dem diese ihre maximale Querabmessung aufweist.

Im einfachsten Fall besteht dazu die Elektrode aus einem Grundmaterial, auf das als Wärmeableiteinrichtung ein wärmeleitender Überzug in Form einer wärmeleitenden Beschichtung, aufgebracht ist. Diese Schicht erstreckt sich vorzugsweise bis zu der Spitze der Elektrode und über große Teile der Flachseiten oder über die gesamten Flachseiten der Elektrode. Die Beschichtung kann sich auch über die Schmalseiten der Elektrode erstrecken. Ist die Elektrode beispielsweise aus Edelstahl oder einem anderen weniger gut wärmeleitenden Material ausgebildet, ist die Wärmeableiteinrichtung aus einem besonders gut wärmeleitenden Material, wie beispielsweise Silber, kohlenstoffähnlichem Diamant (CLD) oder dergleichen ausgebildet. Vorzugsweise aber ist die Wärmeableiteinrichtung aus einem metallischen Material ausgebildet, das auch stromleitend ist, sodass die Wärmeableiteinrichtung zum Beispiel in Gestalt der wärmeableitenden Schicht zur Stromleitung beiträgt und in direktem Kontakt mit dem Entladungsfußpunkt kommen kann. Vorzugsweise ist die Wärmeableiteinrichtung, die z.B. als wärmeleitende Beschichtung ausgebildet ist, auch besonders gut stromleitend. Insbesondere ist es vorteilhaft, wenn die elektrische Leitfähigkeit der Beschichtung größer ist als die elektrische Leitfähigkeit des Grundmaterials.

Bei einer besonders bevorzugten Ausführungsform weist die Wärmeableiteinrichtung somit eine elektrische Leitfähigkeit und insbesondere auch eine thermische Leitfähigkeit auf, die jeweils größer sind als die elektrische und thermische Leitfähigkeit des Grundkörpers.

Bei Beaufschlagung der Elektrode mit hochfrequenter Wechselspannung kann sich dabei der Stromfluss auf die elektrisch hochleitfähige Beschichtung konzentrieren, wobei die ohmschen Leitungsverluste aufgrund der hohen Oberflächenleitfähigkeit gering sind. Damit wird die Wärmeerzeugung an der Elektrode durch ohmsche Verluste reduziert. Die Reduktion der Wärmeerzeugung trägt zur Verlängerung der Standzeit der Elektrode und Verminderung des Materialabtrags wesentlich bei.

In der Zeichnung sind Ausführungsbeispiele der Erfindung veranschaulicht. Es zeigen:
Figur 1 ein erfindungsgemäßes Instrument, das zugehörige speisende Gerät und eine Neutralelektrode, in sehr schematisierter, teilweise perspektivischer Darstellung,
Figur 2 das distale Ende des Instruments nach Figur 1, in perspektivischer schematisierter Längsschnittdarstellung,
Figur 3 die Elektrode des Instruments nach Figur 2, in Seitenansicht,
Figur 4, 5 und 6 verschiedene Querschnitte der Elektrode nach Figur 3,
Figur 7 das Instrument nach den Figuren 2 bis 6, in geschnittener perspektivischer Darstellung während des Betriebs,
Figur 8 eine abgewandelte Ausführungsform einer Elektrode für ein Instrument gemäß Figur 2.

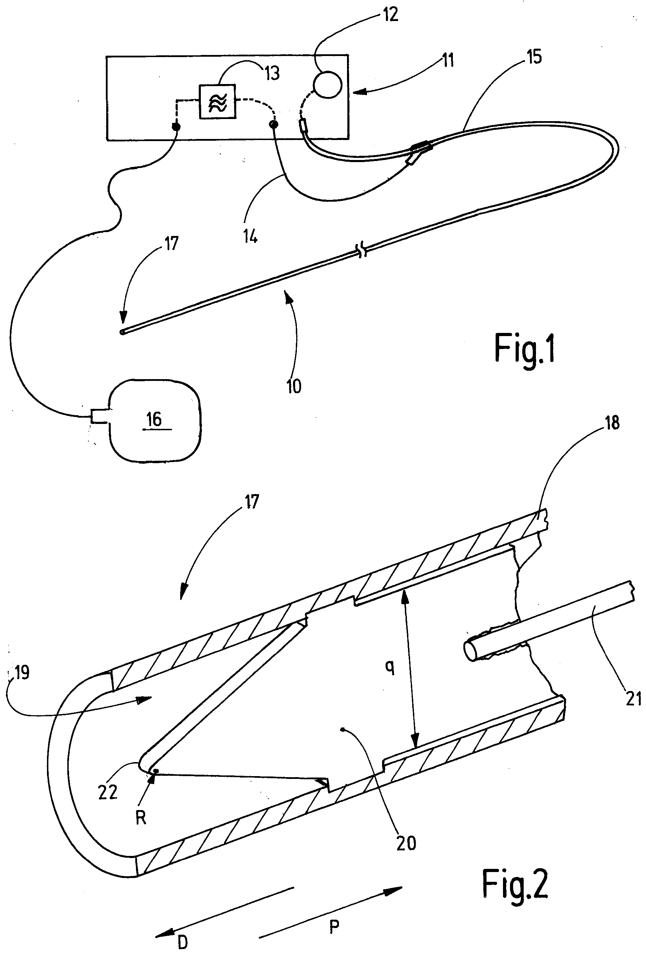

In Figur 1 ist ein Instrument 10 veranschaulicht, das zur plasmagestützten Gewebebehandlung dient. Die Gewebebehandlung kann die Ablation, Koagulation, das Schneiden oder anderweitige Behandlungsarten umfassen.

Das Instrument 10 ist an ein Gerät 11 angeschlossen, das eine Gasquelle 12, zum Beispiel eine Argonquelle, sowie einen Generator 13 zur elektrischen Speisung des Instruments 10 enthält. Diese ist über entsprechende Anschlussmittel mit einer Leitung 14 verbunden, die zu dem Instrument 10 führt und die in eine Leitung 15 eingeführt ist, über die das Instrument 10 mit Gas gespeist wird. Der Generator 13 ist außerdem über entsprechende Verbindungsmittel mit einer Neutralelektrode 16 verbunden, die vor Benutzung des Instruments 10 an dem Patienten anzubringen ist. Die nachstehende Beschreibung gilt aber auch für Instrumente mit anderer Neutralelektrodenkonfiguration.

Das Instrument 10 weist ein distales Ende 17 auf, das gesondert in Figur 2 veranschaulicht ist. Wie ersichtlich gehört zu dem Instrument 10 ein Rohr oder Schlauch 18, das bzw. der ein Lumen 19 umgibt, das am distalen Ende 17 des Schlauchs 18 offen ist. Im Bereich des distalen Endes 17 kann der Schlauch 18 mit einer inneren oder äußeren Verstärkung ersehen sein, beispielsweise in Gestalt einer Keramikhülse, was in Figur 2 nicht näher dargestellt ist. Der Schlauch 18 kann somit ein- oder mehrschichtig aufgebaut sein. Beispiele für Instrumente mit in das offene Ende des Schlauchs 18 eingesetzter Keramikhülse sind der WO 2005/046495 A1 zu entnehmen.

In dem Lumen 19 ist eine Elektrode 20 angeordnet, die mit einem zu der Leitung 14 gehörenden Draht 21 elektrisch verbunden ist, der sich durch das Lumen 19 erstreckt. Der Draht 21 kann mit der Elektrode 20 verschweißt oder auch mechanisch, beispielsweise durch Krimpen, verbunden sein.

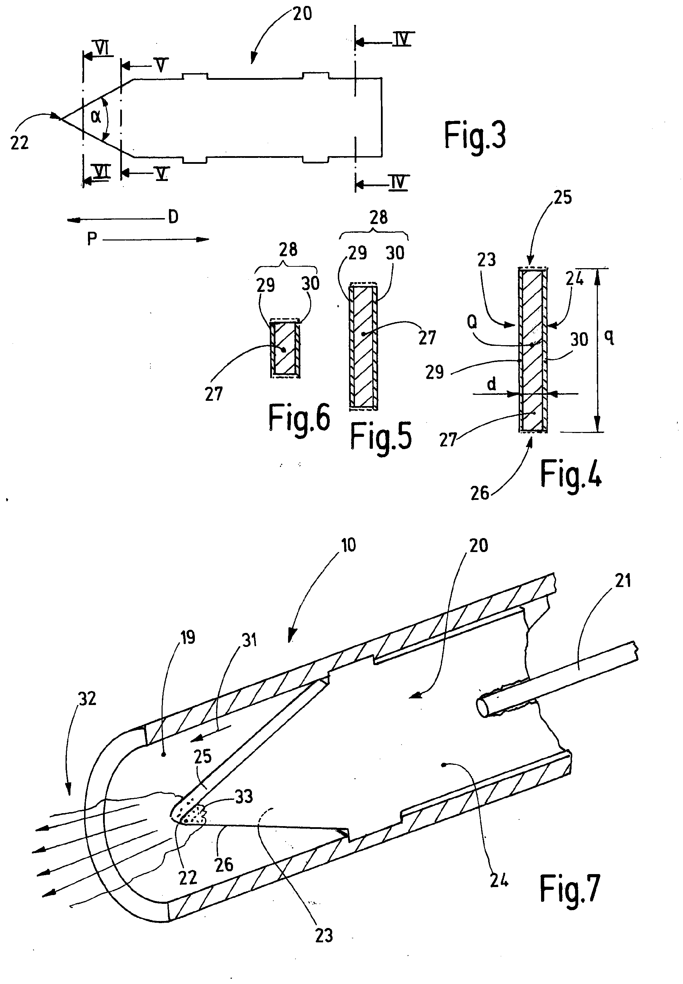

Die Elektrode 20 weist vorzugsweise die in Figur 3 veranschaulichte Grundform auf. An ihrem distalen Ende ist an der Elektrode 20 eine scharfe oder allenfalls leicht gerundete Spitze 22 ausgebildet, deren Rundungsradius R (Figur 2) möglichst gering und vorzugsweise geringer als ein Zehntel der Querabmessung q ist, die quer zur Axialrichtung zu messen ist und mit dem Innendurchmesser des Lumens 19 weitgehend übereinstimmt. Die Elektrode 20 ist vorzugsweise plattenförmig ausgebildet, d.h., ihre Dicke ist wesentlich geringer als ihre Querabmessung q. Dies ergibt sich zum Beispiel aus Figur 4, die den Querschnitt der Elektrode 20 an der in Figur 3 strichpunktierten Schnittlinie IV-IV zeigt. Die Dicke d ist geringer als 1/5 vorzugsweise geringer als 1/10 der Querabmessung q.

Wie weiter aus Figur 4 ersichtlich, weist die Elektrode 20 zwei Flachseiten 23, 24, die durch Schmalseiten 25, 26 verbunden sind. Es ergibt sich somit ein insgesamt viereckiger, vorzugsweise rechteckiger Querschnitt Q, der von den Flachseiten 23, 24 und den Schmalseiten 25, 26 umgrenzt ist. Der viereckige Querschnitt kann auch ein oder mehrfach, z.B. s-förmig gebogen sein.

Die Elektrode 20 weist an ihrem distalen Ende einen Verjüngungsbereich auf, in dem die ansonsten parallel zueinander verlaufenden Schmalseiten 25, 26 zu der Spitze 22 hin konvergent angeordnet sind. Die konvergent aufeinander zu verlaufenden Abschnitte der Schmalseiten 25, 26 können, wie Figur 3 zeigt, gerade oder auch konvex oder konkav ausgebildet sein. Sie legen zwischen einander einen Winkel α fest, der vorzugsweise im Bereich von 20° und 100° liegt.

Wie die Querschnitte V-V und VI-VI zeigen, die gesondert in den Figuren 5 und 6 dargestellt sind, nimmt der Querschnitt der Elektrode 20 zur Spitze 22 hin in distaler Richtung D ab oder mit anderen Worten in proximaler Richtung P zu. Dabei kann die Dicke d der Elektrode 20 im Verjüngungsbereich zur Spitze 22 hin wie die Figuren 5 und 6 zeigen konstant bleiben. Die Dicke d kann jedoch zu der Spitze 22 hin auch abnehmen. Jedenfalls nimmt aber die Querabmessung Q in dem Verjüngungsbereich zur Spitze 22 hin ab.

In einer ersten Ausführungsform besteht die Elektrode 20 insgesamt aus einem gut wärmeleitfähigen Material, wie beispielsweise Wolfram, einem Hartmetall, Kupfer, Aluminium oder einer Kombination dieser Materialien. Genutzt werden können dabei Metalle und auch nichtmetallische elektrisch leitende Materialien wie beispielsweise DLC oder Kombinationen aus Metall und solchen Materialien. Jedenfalls aber hat das verwendete Material dann eine Wärmeleitfähigkeit λ, die größer vorzugsweise deutlich größer ist als die Wärmeleitfähigkeit von Edelstahl. Insbesondere ist λ ≥ 50W/(m*K), ≥ 100W/(m*K), ≥ 200W/(m*K), ≥ 300W/(m*K), ≥ 400W/ (m*K) .

Bei einer bevorzugten Ausführungsform weist die Elektrode 20 einen Mehrschichtaufbau auf, wie er aus den Figuren 4 und 6 hervorgeht. Die Elektrode 20 weist dazu einen Elektrodengrundkörper 27 auf, der zumindest an seinen Flachseiten 23, 24 jedoch optional auch an seinen Schmalseiten 25, 26 mit einer Wärmeableiteinrichtung 28 verbunden ist. Diese besteht im vorliegenden Ausführungsbeispiel aus einer vollflächigen Beschichtung der Flachseiten des Elektrodengrundkörpers 27 mit thermisch leitenden Überzügen 29, 30. Im Ausführungsbeispiel kann der Grundkörper 27 aus Edelstahl bestehen, während die Überzüge 29, 30 aus einem anderen Material mit besserer Wärmeleitfähigkeit und/oder besserer elektrischer Leitfähigkeit bestehen. Als besonders geeignet hat sich dafür Silber erwiesen. Mögliche andere Überzüge besehen aus Aluminium und/oder Kupfer und/oder Hartmetall und/oder DLC und/oder Wolfram und oder einer Schicht, z.B. Metallschicht mit darin eingebettetem CBN (kubisches Bornitrid), Diamantpulver oder ähnlich gut wärmeleitendem Material.

Das insoweit beschriebene Instrument arbeitet wie folgt:
Wie in Figur 7 veranschaulicht, wird das Lumen 19 des Instruments 10 in Betrieb von einem Gasstrom 31 durchflossen, der von der Gasquelle 12 herrührt. Dieser Gasstrom (vorzugsweise Argonstrom) stricht entlang beider Flachseiten 23, 24 der Elektrode 20. Über den Draht 21 wird die Elektrode 20 zugleich mit hochfrequentem elektrischen Strom gespeist. Die Arbeitsfrequenz des Generators 13 und somit die Frequenz des Stroms liegt dabei vorzugsweise oberhalb von 100 kHz, vorzugsweise oberhalb von 300 kHz, weiter vorzugsweise oberhalb von 500 kHz. An der Spitze 22 und einem angrenzendem Bereich derselben verlässt der Strom die Elektrode 20 und bildet einen zu dem nicht weiter veranschaulichten biologischen Gewebe des Patienten überspringenden Funken oder ein dorthin fließendes Plasma 32. Der Fußpunkt 33 des Funkens oder Plasmas berührt dabei die Schmalseiten 25, 26, insbesondere aber die Flachseiten 23, 24 der Elektrode 20, wobei dieser Fußpunktbereich 33 wenigstens 1/10 der axialen (in Proximalrichtung zu messenden) Länge desjenigen Bereichs der Elektrode 20 einnimmt, in dem die Schmalseiten 25, 26 von der Spitze 22 weg divergieren. Die Überzüge 29, 30 erstrecken sich in diesen Bereich hinein und vorzugsweise bis zur Spitze 22. Damit wird der Funken bzw. Plasmastrom elektrisch direkt von dem Überzug 29, 30 gespeist. Die Dicke der Überzüge 29, 30 kann relativ gering sein. Es hat sich gezeigt, dass schon Beschichtungen von 10 bis 20 µm Dicke zu einer wesentlichen Verlängerung der Standzeit der Elektrode 20 und zu einem wesentlich verringerten Materialabtrag von derselben führen. Vorzugsweise beträgt die Dicke der beispielsweise aus Silber bestehenden Überzüge 20 µm, 30 µm oder 50 µm, womit sich bei einer Dicke der Elektrode von z.B. 0,1 mm ein Wärmewiderstand von über 400W/(m*K) ergibt. Die aus der Materialkombination Edelstahl/Silber bestehende Elektrode 20 weist dadurch eine überragende Standzeit auf.

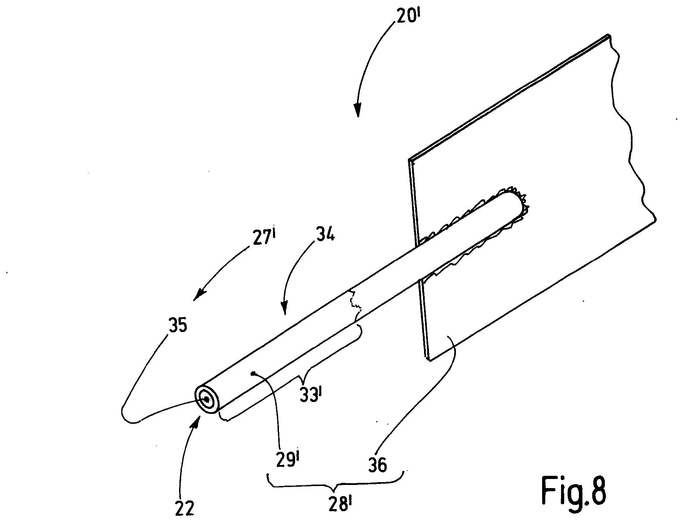

Es ist bei einer abgewandelten Ausführungsform auch möglich, den Elektrodenquerschnitt, anders als bei den vorstehend beschriebenen Ausführungsformen, in proximaler Richtung nicht kontinuierlich, sondern sprunghaft, d.h. in einer oder in mehreren Stufen zunehmen zu lassen. Eine solche Ausführungsform ist in Figur 8 veranschaulicht. Jedoch verwirklicht auch diese Ausführungsform das erfindungsgemäße Konzept, weswegen bei der Beschreibung dieser Elektrode 20' auf die Beschreibung der Ausführungsform nach Figur 1 bis 7 verwiesen wird. Die bereits eingeführten Bezugszeichen werden weitergeführt, wobei diese zur Unterscheidung jeweils mit einem Apostroph versehen sind. Die vorstehende Beschreibung gilt mit Ausnahme der nachfolgenden Besonderheiten entsprechend für die Ausführungsform nach Figur 8.

Die Elektrode 20' weist eine Spitze 22' auf, die hier durch das spitze oder stumpfe Ende eines drahtförmigen Elektrodenabschnitts gebildet sein kann. Dieser drahtförmige Elektrodenabschnitt 34 enthält eine Seele 35, die den Grundkörper 27' bildet und ihrerseits als dünner Zylinderstift ausgebildet sein kann. Die Seele 35 ist mit einem Überzug 29' versehen, der hier, gegebenenfalls in Verbindung mit einem Elektrodenhalteplättchen 36, die Wärmeableiteinrichtung 28' bildet. Der Elektrodenabschnitt 34 kann mit dem Elektrodenhalteplättchen 36 verschweißt, verkrimpt oder anderweitig verbunden sein. Eine stoffschlüssige Verbindung wird wegen der besseren Wärmeübergabe bevorzugt. Der Elektrodenhalteabschnitt 36 kann aus Edelstahl oder einem anderen Material bestehen, das mit einer wärmeleitfähigen Beschichtung, wie Wolfram, Kupfer, Aluminium, DLC oder ähnlichem, versehen oder aus wärmeleitfähigen Material, wie Wolfram, Kupfer, Aluminium, DLC oder ähnlichem, ausgebildet ist.

In Betrieb eines Instruments 10 mit einer Elektrode 20' nach Figur 8 geht eine elektrische Entladung und somit der sich ausbildende Funken- oder Plasmastrom zunächst von der Spitze 22 und dann von zumindest einem Teil des drahtförmigen Elektrodenabschnitts 34 aus. Der elektrisch und thermisch leitfähige Überzug 29', der vorzugsweise eine Silberbeschichtung ist, setzt den elektrischen Widerstand der Elektrode 20 bzw. 20' spürbar herab. Der hochfrequente Wechselstrom des Generators 13 konzentriert sich in den äußeren Schichten der Elektrode 20, 20' und durchfließt so im Wesentlichen den Überzug 29, 30, 29'. Damit werden die ohmschen Verluste an der Elektrode 20, 20' minimiert und zudem wird die reduzierte Wärmemenge durch den Überzug 29, 30 wesentlich besser von dem Entladungsfuß weg geleitet und so verteilt, dass sie großflächig auf dem Gasstrom übertragen werden kann.

Bei einem verbesserten Instrument 10 ist die Elektrode 20, 20' mit einer Wärmeableiteinrichtung 28, 28' versehen, sodass der Wärmewiderstand der Elektrode 20, 20' gemessen in Längsrichtung (in distaler oder proximale Richtung) vorzugsweise ≥ 300W/(m*K) ist. Bei einer bevorzugten Ausführungsform ist die Wärmeableiteinrichtung 28, 28' durch einen Überzug 29, 30, 29' gebildet, der gegenüber dem Material des Elektrodengrundkörpers 27, 27' eine höhere elektrische Leitfähigkeit wie auch eine höhere thermische Leitfähigkeit aufweist.

### Bezugszeichen:

- 10: Instrument
- 11: Gerät
- 12: Gasquelle
- 13: Generator
- 14: Leitung (für Strom)
- 15: Leitung (für Gas)
- 16: Neutralelektrode
- 17: distales Ende des Instruments 10
- 18: Schlauch
- 19: Lumen
- 20: Elektrode
- 21: Draht
- 22: Spitze
- q: Querabmessung der Elektrode 20
- d: Dicke der Elektrode 20
- 23, 24: Flachseiten der Elektrode 20
- 25, 26: Schmalseiten der Elektrode 20
- Q: Querschnitt der Elektrode 20
- α: Winkel zwischen Abschnitten der Schmalseiten 25,26
- D: Distale Richtung
- P: Proximale Richtung
- λ: Wärmeleitfähigkeit
- 27: Elektroden-Grundkörper
- 28: Wärmeableiteinrichtung
- 29, 30: Überzüge
- 31: Gasstrom
- 32: Plasma
- 33: Fußpunkt
- 34: drahtförmiger Elektrodenabschnitt
- 35: Seele
- 36: Elektrodenhalteabschnitt

## Patentansprüche

1. Elektrodenanordnung für ein elektrochirurgisches Instrument (10), insbesondere zur Plasmakoagulation, mit einer Elektrode (20, 20'), die eine distal orientierte Spitze (22) aufweist, von der ausgehend der Elektrodenquerschnitt (Q) durch eine entlang der Axialrichtung in proximaler Richtung von der Spitze (22) weg zunehmende Querabmessung (q) der Elektrode (20) in proximaler Richtung (P) zunehmend ausgebildet ist, wobei die Elektrode (20, 20') aus einem Grundkörper (27, 27') und einem auf einer Fläche des Grundkörpers angeordneten wärmeleitenden Überzug (29, 30) besteht,
wobei die Elektrode (20, 20') aus einer Materialkombination besteht, die eine Wärmeleitfähigkeit (λ) aufweist, die größer ist als 20 W/(m*K),
wobei die Elektrode (20) als Plättchen ausgebildet ist, das zwei Flachseiten (23, 24) aufweist, die durch Schmalseiten (25, 26) miteinander verbunden sind,
wobei zwischen den Schmalseiten (25, 26) und den Flachseiten (23, 24) Kanten ausgebildet sind.

2. Elektrodenanordnung nach Anspruch 1, wobei die Elektrode (20, 20') eine maximale Querabmessung (q) und an ihrer Spitze (22) einen Rundungsradius (R) aufweist, der kleiner als ein Zehntel der maximalen Querabmessung (q).

3. Elektrodenanordnung nach Anspruch 1, wobei die Querabmessung (q) in proximaler Richtung von der Spitze (22) ausgehend kontinuierlich zunehmend ausgebildet ist.

4. Elektrodenanordnung nach einem der vorstehenden Ansprüche, wobei die Elektrode (20) ausgehend von der Spitze (22) stufenlos ausgebildete Kanten aufweist.

5. Elektrodenanordnung nach Anspruch 1, wobei die Elektrode (20, 20') mindestens eine Flachseite (23, 24) aufweist und wobei der Überzug die gesamte Flachseite (23, 24) einnehmend ausgebildet ist.

6. Elektrodenanordnung nach einem der vorstehenden Ansprüche , wobei der Überzug aus einem metallischen Material ausgebildet ist.

7. Elektrodenanordnung nach einem der vorstehenden Ansprüche , wobei der Überzug aus einem nichtmetallischen Material ausgebildet ist.

8. Elektrodenanordnung nach einem der vorstehenden Ansprüche, wobei der Überzug sich bis in einen Bereich (33) erstreckend ausgebildet und angeordnet ist, der zum direkten Kontakt mit einem von der Elektrode (20, 20') ausgehenden Funken vorgesehen ist.

9. Elektrodenanordnung nach einem der vorstehenden Ansprüche, wobei der Überzug eine elektrische Leitfähigkeit aufweist, die größer ist als die elektrische Leitfähigkeit des Grundkörpers (27, 27').

10. Elektrodenanordnung nach einem der vorstehenden Ansprüche, wobei der Überzug eine thermische Leitfähigkeit aufweist, die größer ist als die thermische Leitfähigkeit des Grundkörpers (27, 27').

## Claims

1. Electrode arrangement for an electrosurgical instrument (10), particularly for plasma coagulation,
having an electrode (20, 20') that comprises a tip (22) orientated in distal direction, starting therefrom the electrode cross-section (Q) is configured to increase in proximal direction (P) by a transverse dimension (q) that increases from the tip (22) in proximal direction, wherein the electrode (20, 20') consists of a base body (27, 27') an a thermally conductive overlay (29, 30) provided on a surface of the base body,
wherein the electrode (20, 20') consists of a material combination having a thermal conductivity (λ) that is larger than 20 W/(m*K),
wherein the electrode (20, 20') is configured as platelet that comprises two flat sides (23, 24) that are connected with each other by narrow sides (25, 26),
wherein edges are formed between the narrow sides (25, 26) and the flat sides (23, 24).

2. Electrode arrangement according to claim 1, wherein the electrode (20, 20') has a maximum transverse dimension (q) and a radius of curvature (R) at its tip (22) that is smaller than 1/10 of the maximum transverse dimension (q).

3. Electrode arrangement according to claim 1, wherein the transverse dimension (q) is configured to continuously increase in proximal direction starting from the tip (22).

4. Electrode arrangement according to any of the preceding claims, wherein the electrode (20) comprises stepless configured edges starting from the tip (22).

5. Electrode arrangement according to any of the preceding claims, wherein the electrode (20) comprises at least one flat side (23, 24) and wherein the overlay is configured to cover the entire flat side (23, 24).

6. Electrode arrangement according to any of the preceding claims, wherein the overlay is made of a metallic material.

7. Electrode arrangement according to any of the preceding claims, wherein the overlay is made of a non-metallic material.

8. Electrode arrangement according to any of the preceding claims, wherein the overlay is configured and arranged to extend up to an area (33) that is provided for direct contact with a spark originating from the electrode (20, 20').

9. Electrode arrangement according to any of the preceding claims, wherein the overlay comprises an electrical conductivity that is larger than the electrical conductivity of the base body (27, 27').

10. Electrode arrangement according to any of the preceding claims, wherein the overlay has a thermal conductivity that is larger than the thermal conductivity of the base body (27, 27').

## Revendications

1. Ensemble d'électrodes pour un instrument électrochirurgical (10), en particulier pour la coagulation plasmatique, avec une électrode (20, 20'), qui présente une pointe (22) orientée distalement, à partir de laquelle la section transversale d'électrode (Q) est réalisée par une dimension transversale (q) de l'électrode (20) augmentant le long de la direction axiale vers la direction proximale à partir de la pointe (22) dans la direction proximale (P), dans lequel l'électrode (20, 20') est constituée d'un corps de base (27, 27') et d'un revêtement thermoconducteur (29, 30) disposé sur une surface du corps de base, dans lequel l'électrode (20, 20') est constituée d'une combinaison de matériaux qui présente une conductivité thermique (λ) qui est supérieure à 20 W/(m*K), dans lequel l'électrode (20) est réalisée sous forme d'une plaquette, qui présente deux faces plates (23, 24) reliées entre elles par des faces étroites (25, 26), dans lequel des bords sont réalisés entre les faces étroites (25, 26) et les faces plates (23, 24).

2. Ensemble d'électrodes selon la revendication 1,
dans lequel l'électrode (20, 20') présente une dimension transversale maximale (q) et sur son extrémité (22) un rayon d'arrondi (R) qui est inférieur à un dixième de la dimension transversale maximale (q).

3. Ensemble d'électrodes selon la revendication 1, dans lequel la dimension transversale (q) est réalisée de manière à augmenter en continu dans la direction proximale à partir de la pointe (22).

4. Ensemble d'électrodes selon l'une quelconque des revendications précédentes, dans lequel l'électrode (20) présente des bords réalisés en continu à partir de la pointe (22).

5. Ensemble d'électrodes selon la revendication 1, dans lequel l'électrode (20, 20') présente au moins une face plate (23, 24) et dans lequel le revêtement est réalisé de manière à occuper l'ensemble de la face plate (23, 24).

6. Ensemble d'électrodes selon l'une des revendications précédentes, dans lequel le revêtement est réalisé à partir d'un matériau métallique.

7. Ensemble d'électrodes selon l'une quelconque des revendications précédentes, dans lequel le revêtement est réalisé à partir d'un matériau non métallique.

8. Ensemble d'électrodes selon l'une quelconque des revendications précédentes, dans lequel le revêtement est réalisé et disposé de manière à s'étendre jusqu'à une zone (33) qui est prévue pour le contact direct avec une étincelle émanant de l'électrode (20, 20').

9. Ensemble d'électrodes selon l'une quelconque des revendications précédentes, dans lequel le revêtement présente une conductivité électrique qui est supérieure à la conductivité électrique du corps de base (27, 27').

10. Ensemble d'électrodes selon l'une quelconque des revendications précédentes, dans lequel le revêtement présente une conductivité thermique qui est supérieure à la conductivité thermique du corps de base (27, 27').
